# EUROPÄISCHE PATENTANMELDUNG

(11) **EP 3 141 269 A1**
(43) Veröffentlichungstag der Anmeldung: **15.03.2017**
(21) Anmeldenummer: 15184794.4
(22) Anmeldetag: 11.09.2015
(51) Int. Cl.: A61M 1/10

(54) **SYSTEM ZUM VERBINDEN EINER BLUTPUMPE MIT EINEM HERZEN**

(71) Anmelder: Berlin Heart GmbH, 12247 Berlin (DE)
(72) Erfinder: ARSLAN, Nedim, 10717 Berlin (DE); GUNDLACH, Heiko, 10435 Berlin (DE); KAEBE, Benjamin Daniel, Scullin ACT 2614 (AU); LAUTERBACH, Gerhard, 12679 Berlin (DE); MATTHES, Michael, 15345 Altlandsberg (DE); WINTERWERBER, Kim Peter, 12357 Berlin (DE)
(74) Vertreter: Pfenning, Meinig & Partner mbB

(57) **Zusammenfassung**

Die vorliegende Offenbarung betrifft ein System zum Verbinden einer Blutpumpe mit einem Herzen, umfassend: eine Blutpumpe zur Förderung von Blut, wobei die Blutpumpe einen ersten rohrförmigen Abschnitt und einen zweiten rohrförmigen Abschnitt umfasst und zwischen dem ersten und dem zweiten rohrförmigen Abschnitt ein flanschförmiger Abschnitt vorhanden ist; und einen Konnektor mit einem sich zwischen einem distalen Ende und einem proximalen Ende in einer axialen Richtung erstreckenden rohrförmigen Konnektorabschnitt mit einem Lumen zur Aufnahme des ersten rohrförmigen Abschnitts der Blutpumpe und mit einem am distalen Ende angeordneten flanschförmigen Konnektorabschnitt zur Befestigung des Konnektors an einem Organ, wobei eine das Lumen umlaufende Wand des ersten rohrförmigen Konnektorabschnitts eine die Wand zumindest abschnittsweise umlaufende Aufweitung mit einer Rastnase aufweist. Im flanschförmigen Abschnitt der Blutpumpe ist ein in einem Führungsspalt aufweitbarer Sperrring angeordnet, welcher derart im Führungsspalt geführt und konfiguriert ist, dass der Sperrring in einem ersten Zustand die Aufweitung der Wand hintergreift und in einem zweiten, aufgeweiteten Zustand die Wand nicht hintergreift.

## Beschreibung

Gegenstand der vorliegenden Anmeldung ist ein System zum Verbinden einer Blutpumpe mit einem Herzen, das eine Blutpumpe sowie einen Konnektor, der an einem Organ, wie beispielsweise einem Herzen, befestigt werden kann und in den die Blutpumpe eingeschoben werden kann, umfasst.

Herzunterstützungspumpen, wie beispielsweise Ventricular Assist Devices (VAD), sind im Stand der Technik hinlänglich bekannt. Zudem existieren zahlreiche Vorschläge, wie derartige Pumpen mit einem Organ, wie beispielsweise mit einem Herzen, verbunden werden können.

Eine Vielzahl der VAD-Konnektor-Systeme umfasst einen Konnektor, der beispielsweise an einem Apex eines linken Ventrikels, beispielsweise mithilfe einer Naht oder einer Spiralfeder, verankert wird. Anschließend wird in den Ventrikel eine Öffnung geschnitten, durch welche die Blutpumpe in den Ventrikel geschoben werden kann.

Um eine sichere Befestigung der Blutpumpe mit dem Konnektor zu gewährleisten, sind im Stand der Technik vielerlei Systeme bekannt. So zeigt beispielsweise die US 6 802 806 B2 ein VAD-Konnektor-System, bei dem die Herzpumpe bzw. eine Kanüle einer Herzpumpe mit einem Konnektor verschraubt werden kann. Obgleich die Verschraubung eine sichere Befestigung erlaubt, hat diese den Nachteil, dass das Schrauben, beispielsweise im menschlichen Körper, mit Schwierigkeiten verbunden ist.

Eine weitere Lösung ist beispielsweise in der US 8403 823 B2 dargestellt, die vorschlägt, einen Einlassstutzen einer Blutpumpe mit einem Konnektor mittels Stiften bzw. Schrauben zu verbinden. Obgleich auch hier eine sichere Verbindung gewährleistet werden kann, ist das Einbringen der Schrauben im Körper schwierig.

Ein weiteres System ist in der US 8152845 B2 dargestellt, die einen Nahtring mit einer daran befestigten Manschette vorschlägt, welche über einen rohrförmigen Abschnitt einer Blutpumpe gestülpt werden kann und welche mit der Blutpumpe entweder mittels Spannkraft sicher befestigt oder welche mit der Blutpumpe vernähbar ist. Obgleich auch hier eine sichere Befestigung gewährleistet werden kann, ist das Befestigen der Blutpumpe an der Manschette im Körper mit Schwierigkeiten verbunden.

Ein weiteres System ist in der US 7 942 805 B2 dargestellt. Das System umfasst einen Konnektor, der einen nach innen wirkenden Sperrring umfasst, der in eine Nut eines Einlassstutzens radial eingreifen kann. Der Sperrring wird hierbei mittels einer Schraube radial verengt, so dass der Ring des Konnektors die Nut des Einlassstutzens der Blutpumpe hintergreift und axial fixiert. Obgleich hiermit eine sichere Befestigung möglich ist, ist das Bedienen der Schraube im Körper mit Schwierigkeiten verbunden. Zudem bedarf es einer Nut am Einlassstutzen der Pumpe, die hintergriffen werden muss.

Ein weiteres System ist aus der EP 2 5247 09 A1 bekannt. Das System umfasst einen Nahtring, der unter anderem einen Ringkörper mit einer Nut aufweist. An der Blutpumpe sind zwei Frontplatten mit einem zwischen den Frontplatten angeordneten Federkörper angeordnet. Der Federkörper ist dabei derart geformt, dass er in einer ersten Konfiguration auf den Ringkörper geschoben werden kann und in einer zweiten Konfiguration gespannt werden kann, so dass die Nut des Ringkörpers hintergriffen wird. Der Federkörper ist derart konfiguriert, dass er in einem Führungskörper gehalten ist und aufgespreizt werden kann, um über den Ringkörper geführt zu werden, und anschließend der zur Spreizung aufgebrachte Druck gelöst wird, so dass der Federkörper die Nut des Ringkörpers hintergreift. Hierdurch wird ein sicheres Hintergreifen ermöglicht, jedoch kann die Handhabung nicht unabhängig von der radialen Position der Blutpumpe durchgeführt werden.

Aufgabe der vorliegenden Erfindung ist es daher, ein VAD-Konnektor-System vorzuschlagen, das eine Alternative zu den bereits bestehenden Systemen bietet.

Gelöst wird die Aufgabe gemäß einem System nach Anspruch 1 sowie den unter- und nebengeordneten Ansprüchen. Die hier behandelten Pumpen können beispielsweise Links-VADs (LVADs), Rechts-VADs (RVADs) oder beidseitige-VADs (BiVADs) sein.

Anmeldungsgemäß umfasst die Blutpumpe zur Förderung von Blut einen ersten rohrförmigen Abschnitt, beispielsweise einen Einlassstutzen, und einen zweiten rohrförmigen Abschnitt, beispielsweise einen Auslassbereich einer Pumpe, wobei zwischen dem ersten und zweiten rohrförmigen Abschnitt ein flanschförmiger Abschnitt angeordnet ist. Im flanschförmigen Abschnitt der Blutpumpe ist ein in einem Führungsspalt aufweitbarer Sperrring angeordnet, wobei der Sperrring bevorzugt radial aufweitbar ist. Der Sperrring ist so im Führungsspalt geführt und konfiguriert, dass er in einem ersten, unbelasteten Zustand einen ersten Durchmesser und in einem zweiten, aufgeweiteten Zustand einen zweiten Durchmesser besitzt, wobei der zweite Durchmesser größer als der erste Durchmesser ist.

Das System umfasst ferner einen Konnektor mit einem sich zwischen einem distalen Ende und einem proximalen Ende in einer axialen Richtung erstreckenden rohrförmigen Konnektorabschnitt mit einem Lumen zur Aufnahme des ersten rohrförmigen Abschnitts der Blutpumpe und mit einem am distalen Ende angeordneten flanschförmigen Konnektorabschnitt zur Befestigung des Konnektors an einem Organ, wie beispielsweise einem Herzen. Im ersten rohrförmigen Konnektorabschnitt ist eine das Lumen umlaufende Wand vorhanden, die zumindest abschnittsweise eine Aufweitung aufweist, die in Form einer Rastnase ausgebildet ist.

Die rastnasenförmige Aufweitung weitet sich vom proximalen zum distalen Ende hin auf und umfasst an ihrem distalen Ende eine zu hintergreifende Fläche. Wird die Blutpumpe mit dem ersten rohrförmigen Abschnitt in axialer Richtung in das Lumen eingeschoben, trifft der Sperrring auf das proximale Ende der rastnasenförmigen Aufweitung und wird aufgrund des Profils der Aufweitung beim weiteren Aufschieben der Blutpumpe zum distalen Ende des Konnektors hin weiter aufgeweitet, bis die Rastnase ihre maximale radiale Ausdehnung erreicht hat, und schnappt anschließend auf die zu hintergreifende Fläche und hintergreift diese. Da der Sperrring zunächst langsam aufgeweitet wird und anschließend "einschnappt", kommt es zu einem beispielsweise klickenden Geräusch, welches der die Blutpumpe einführenden Person ein hörbares Feedback gibt, dass die Blutpumpe sicher auf dem Konnektor angeordnet ist. Zudem können durch das Einschnappen Vibrationen im Pumpengehäuse induziert werden, welche ein taktiles Feedback vermitteln.

Da der Sperrring durch die Rastnase aufgeweitet wird, ohne dass zusätzlicher Druck den Sperrring weiten muss, ist der die Blutpumpe einsetzende Arzt freier in seiner Handhabung der Pumpe, da er sich nur mit dem Aufschieben beschäftigen muss. Ein zusätzliches Fixieren mittels Schrauben, Drücken oder Vernähen, wie in einigen Vorrichtungen nach dem Stand der Technik, ist nicht vonnöten. Hierdurch wird eine hohe Sicherheit bei der Operation gewährleistet, da die die Pumpe einsetzende Person die Pumpe nicht an bestimmten Stellen umgreifen, sondern lediglich die Pumpe axial in das Lumen einschieben muss.

Da es sich bei den rohrförmigen Abschnitten zumeist um zylinderförmige Abschnitte handelt, ist es zudem möglich, eine freie radiale Orientierung der Pumpe beim Aufschieben des ersten rohrförmigen Abschnitts in das Lumen zu wählen, die sich an die Anatomie des Körpers anpasst. Eine vorhergehende Ausrichtung des Konnektors zur Blutpumpe ist nicht vonnöten. Da der Sperrring die Rastnase hintergreift und der erste Durchmesser des Sperrrings so gewählt werden kann, dass die Pumpe weiterhin frei rotierbar ist, kann die Blutpumpe nach erfolgtem Hintergreifen der rastnasenförmigen Aufweitung des Konnektors durch den Sperrring frei rotiert werden. Dadurch, dass der Sperrring in einem zweiten, aufgeweiteten Zustand die Wand bzw. die rastnasenförmige Aufweitung nicht (mehr) hintergreift, kann die Pumpe nach Bedarf auch wieder entfernt werden und in axialer Richtung vom Konnektor abgezogen werden.

Der Sperrring greift also auf der Außenseite der Konnektorwand in die Rastnase ein. Auf diese Weise ist es möglich, dass der erste rohrförmige Abschnitt, der in das Lumen des Konnektors geschoben wird, ohne eine Nut ausgebildet werden kann. Zwischen der Innenwand des Konnektors und dem ersten rohrförmigen Abschnitt der Blutpumpe kann ein Dichtring angeordnet werden, der eine fluiddichte Abdichtung zwischen Blutpumpe und Konnektor herstellt. Hierfür kann beispielsweise die Innenwand des Konnektors oder der rohrförmige Abschnitt der Blutpumpe eine Nut mit einem Dichtring aufweisen.

Weitere Ausführungsbeispiele werden nachstehend im Rahmen der jeweiligen Lehre der untergeordneten Ansprüche erläutert.

In einer Ausführungsform ist die rastnasenförmige Aufweitung der Wand so ausgebildet, dass sie die Wand vollständig umläuft. In dieser Ausführungsform ist die rastnasenförmige Aufweitung vorzugsweise eine geschlossene Rastnase, die sich in axialer Richtung vom proximalen zum distalen Ende hin erstreckt. In diesem Ausführungsbeispiel ist es weiterhin vorteilhaft, wenn die Wand das Lumen ebenfalls vollständig umläuft. In anderen Ausführungsbeispielen ist es jedoch möglich, dass die Aufweitung an sich in axialer Richtung erstreckenden Fingern angeordnet ist, die voneinander beabstandet sind. Bei dieser Ausführungsform wird zwar weniger Material verwendet, jedoch ist die Stabilität der Finger nicht so hoch wie eine vollständig umlaufende Wand.

In einer weiteren Ausführungsform ist die Rastnase so konfiguriert, dass sie an einem proximalen Ende einen ebenen Abschnitt umfasst, der mit einem ebenen Bereich des flanschförmigen Abschnitts der Blutpumpe korrespondiert, so dass diese ebenen Bereiche flächig aneinander anliegen. Hierdurch wird ein in axialer Richtung liegender Anschlag gebildet, so dass vermieden wird, dass die Pumpe zu weit in den Konnektor geschoben wird. Zugleich können die ebenen Abschnitte so gewählt sein, dass durch das flächige Aneinanderliegen die Position der Pumpe auf dem Konnektor stabilisiert wird und ein übermäßiges Wackeln der Pumpe verhindert wird.

In einer weiteren Ausführungsform ist der Führungsspalt so konfiguriert, dass er eine Breite besitzt, die mit einer Höhe des Sperrrings, bevorzugt einer axialen Höhe des Sperrrings, korrespondiert, so dass der Sperrring an der Blutpumpe in axialer Richtung fixiert ist. Auf diese Weise kann sichergestellt werden, dass der Sperrring lediglich eine radiale Bewegung, nicht jedoch eine wesentliche axiale Bewegung ausführt. Die Spaltbreite des Führungsspalts ist dabei jedoch so gewählt, dass der Sperrring reibungsarm in dem Führungsspalt radial bewegt werden kann.

In einem weiteren Ausführungsbeispiel sind die Aufweitung und der Sperrring so gewählt, dass die Blutpumpe im ersten, die Rastnase hintergreifenden Zustand um die axiale Richtung drehbar gehalten ist. Auf diese Weise kann die Blutpumpe nach dem sicheren Verbinden mit dem Konnektor in die gewünschte Position gedreht werden.

Alternativ zur drehbaren Lagerung können Blutpumpe und Konnektor derart konfiguriert sein, dass diese im ersten, d. h. ineinandergeführten Zustand drehfest zueinander angeordnet sind. In einer Ausführungsform umfasst die Blutpumpe dabei einen ersten gezahnten Abschnitt. Der Konnektor weist einen zweiten gezahnten Abschnitt auf, wobei die beiden gezahnten Abschnitte miteinander korrespondieren, d. h. die Zähne der jeweiligen Abschnitte ineinander eingreifen können. Während die Zähne ineinandergreifen, sind der Konnektor und die Blutpumpe gegeneinander nicht mehr verdrehbar, d. h., sie sind zueinander drehfest. Um die Blutpumpe gegenüber dem Konnektor zu verdrehen, muss der Sperrring gelöst werden und die Blutpumpe in axialer Richtung aus dem Konnektor gezogen werden. Sobald der gezahnte Abschnitt der Blutpumpe nicht länger in den gezahnten Abschnitt des Konnektors eingreift, kann die Blutpumpe wieder gegenüber dem Konnektor verdreht werden. Sobald die gewünschte Orientierung der Blutpumpe eingestellt ist, wird die Blutpumpe wieder in den Konnektor geschoben, und die gezahnten Abschnitte greifen ineinander ein.

In einer Weiterbildung der gezahnten Abschnitte der Blutpumpe und/oder des Konnektors sind diese in axialer Richtung betrachtet rotationssymmetrisch angeordnet. Hierdurch wird gewährleistet, dass in jeder beliebigen Orientierung der Blutpumpe zum Konnektor eine drehfeste Fixierung möglich ist. In einer Weiterführung sind hierbei beide gezahnten Abschnitte rotationssymmetrisch und korrespondierend zueinander ausgebildet.

In einem weiteren Ausführungsbeispiel ist der Führungsspalt in einer radialen Richtung offen. Auf diese Weise kann der Sperrring bei der Montage der Blutpumpe erst zu einem vergleichsweise späten Zeitpunkt in den Führungsspalt eingeschoben werden. Dieses Einschieben des Sperrrings kann jedoch außerhalb des Körpers vorgenommen werden oder bereits bei der Fertigung der Pumpe geschehen. Zudem dient ein offener Führungsspalt dazu, dass beim Aufweiten des Sperrrings Gewebe, das sich zwischen dem Sperrring und der Pumpe gesammelt hat, aus dem offenen Führungsspalt verdrängt werden und somit der Sperrring auch nach einem längeren Verbleib im Körper weiterhin radial aufgeweitet werden kann. Dies ist beispielsweise bei einem Pumpenwechsel oder bei einer Explantation notwendig.

In einem weiteren Ausführungsbeispiel besitzt der flanschförmige Abschnitt der Blutpumpe einen radialen Anschlag, der verhindert, dass der Sperrring auf einen kleineren Durchmesser als den ersten Durchmesser verengt werden kann. Dies ist insbesondere vor der Implantation hilfreich, da ein Beschädigen des Sperrrings vor der Implantation verhindert werden kann. Der Anschlag kann entweder durch den flanschförmigen Abschnitt selbst gegeben sein oder mithilfe eines weiteren Bauteils, eines Anschlagrings, realisiert werden.

In einem weiteren Ausführungsbeispiel weist der Sperrring ein abgewinkeltes Profil, beispielsweise ein L-förmiges Profil, auf, wobei ein erster Abschnitt des Sperrrings zum Hintergreifen der rastnasenförmigen Aufweitung konfiguriert ist und ein weiterer, zum hintergreifenden Abschnitt abgewinkelter Abschnitt so ausgebildet ist, dass er am Anschlag der Blutpumpe radial anliegt und abgestützt ist. Durch die Abstützung kann ein Verengen des Sperrrings auf einen Durchmesser unterhalb des ersten Durchmessers verhindert werden.

In einem weiteren Ausführungsbeispiel ist der Sperrring ein offener Sperrring und umfasst ein Winkelsegment von beispielsweise mehr als 270°, vorzugsweise mehr als 300°, vorzugsweise mehr als 330°, in einigen Ausführungsbeispielen ein offener überlappender Sperrring mit einem Winkelsegment von mehr als 390°. An einem Ende ist der Sperrring am flanschförmigen Abschnitt der Blutpumpe fixiert, vorzugsweise stoffschlüssig oder formschlüssig fixiert. So kann der Sperrring beispielsweise an der Blutpumpe angeschweißt, in einem Stift drehbar gehalten oder auf ähnliche oder gleichwertige Weise fixiert sein.

In einer weiteren Ausführungsform weist der Sperrring ein Greifelement zur Überführung des Sperrrings vom ersten in den zweiten Zustand auf. Mithilfe eines radial aus dem flanschförmigen Abschnitt der Blutpumpe hervorstehenden Greifelements des Sperrrings kann dieser vom ersten in den zweiten Zustand aufgeweitet werden.

In einer weiteren Ausführungsform ist zum Greifelement korrespondierend an der Blutpumpe ein weiteres Greifelement angeordnet, so dass beim Greifen des Greifelements des Sperrrings und des an der Pumpe aufgebrachten Greifelements und einem anschließenden Zusammenführen der beiden Elemente der Sperrring aufgeweitet wird. Hierdurch wird das Explantieren der Blutpumpe stark vereinfacht. Es ist jedoch zu beachten, dass die Greifelemente lediglich zum Explantieren oder zum Abziehen der Pumpe vonnöten sind. Beim Aufschieben der Pumpe in das Konnektorelement wird der Sperrring durch die Rastnase aufgeweitet. Das Greifelement bzw. das dazu korrespondierende Greifelement der Blutpumpe ist lediglich vonnöten, um eine Aufweitung des Sperrrings von der hintergreifenden Fläche der Rastnase zu bewirken.

Wie bereits erwähnt, kann der Führungsspalt in einer radialen Richtung offen ausgebildet sein. Alternativ oder zusätzlich hierzu kann die Blutpumpe im flanschförmigen Abschnitt oder im zweiten rohrförmigen Abschnitt weitere Öffnungen zur Verdrängung von zwischen dem Sperrring und der Blutpumpe befindlichem Gewebe haben. Hierdurch wird das Explantieren der Pumpe vereinfacht. In einigen Ausführungsformen kann nur eine Öffnung, in anderen Ausführungsformen mehr als eine Öffnung vorgesehen sein.

In einer weiteren Ausführungsform umfasst das System einen auf die Blutpumpe stülpbaren Mantel. "Stülpbar" ist hierbei derart zu verstehen, dass der Mantel beispielsweise flexibel ist und erst später auf die Blutpumpe aufgezogen wird. Der Mantel umfasst mindestens eine, vorzugsweise nach innen ragende Zunge, die in die mindestens eine Öffnung des flanschförmigen Abschnitts der Blutpumpe eingreift. Der Mantel kann beispielsweise aus Silikon oder einem anderen weichen, biokompatiblen Material gefertigt sein. Dadurch, dass die Zunge in die mindestens eine Öffnung ragt, kann verhindert werden, dass die Öffnung durch einwachsendes Gewebe verstopft wird. Bei der Explantation der Blutpumpe kann der Mantel entfernt werden und das wenige, verbliebene Gewebe leicht aus den Öffnungen verdrängt werden. Es wird darauf hingewiesen, dass die Anmelderin den Mantel im Rahmen einer Teilanmeldung eigenständig zur Verwendung mit einem VAD-System weiterverfolgen wird. Selbiges gilt für die gezahnten Abschnitte des Konnektors und der Blutpumpe.

In einer weiteren Ausführungsform umfasst der erste rohrförmige Abschnitt der Blutpumpe eine Nut, in welcher ein Dichtelement, beispielsweise in Form eines Dichtrings, angeordnet ist. Alternativ hierzu kann der Konnektor auf der Innenseite der Wand, die das Lumen umläuft, eine Nut oder einen Dichtring umfassen.

Nachfolgend werden einige Ausführungsbeispiele eines VAD-Konnektor-Systems erläutert. An dieser Stelle sei noch darauf hingewiesen, dass sowohl die Blutpumpe für sich genommen als auch der Konnektor für sich genommen einen eigenständigen Teil der Anmeldung bilden und im Rahmen von weiterführenden Anmeldungen verfolgt werden können.

Es zeigt
- Fig. 1: eine schematische Ansicht einer Pumpe;
- Fig. 2: eine schematische Ansicht eines Konnektors;
- Fign. 3A-C: einen Querschnitt einer Pumpe mit Sperrring;
- Fig. 3D: ein weiteres Ausführungsbeispiel eines Sperrrings;
- Fign. 4A, B: einen Querschnitt eines schematischen Konnektors;
- Fign. 5A-C: verschiedene Längsschnitte durch ein Pumpen-Konnektor-System;
- Fign. 6 A, B: eine weitere Ausführungsform eines Pumpen-Konnektor-Systems;
- Fign. 7A-C: eine weitere Ausführungsform eines Pumpen-Konnektor-Systems; und
- Fig. 8: eine Ausführungsform eines Silikonmantels für das System.

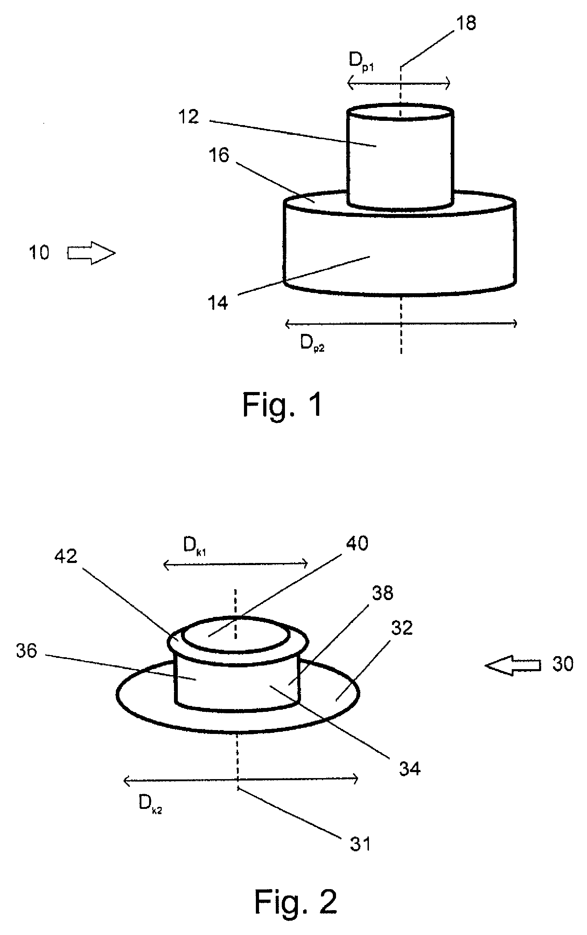

Figur 1 zeigt schematisch eine Blutpumpe 10, welche einen ersten rohrförmigen Abschnitt 12 und einen zweiten rohrförmigen Abschnitt 14 umfasst, die über einen flanschförmigen Abschnitt 16 miteinander gekoppelt sind. Die drei Abschnitte 12, 14 und 16 sind koaxial entlang der Achse 18 ausgerichtet. Der erste rohrförmige Abschnitt 12 kann dabei beispielsweise als Einlassstutzen dienen und kann, je nach verwendetem Konnektorsystem, beispielsweise in einen Ventrikel durch die Herzwand eingeschoben werden. Der zweite rohrförmige Abschnitt 14 umfasst im Inneren beispielsweise eine Spiralkammer und zudem einen hier nicht näher dargestellten Auslass, über welchen das durch den Einlass angesaugte Fluid ausgestoßen wird. In dem vorliegenden Ausführungsbeispiel besitzt der rohrförmige Abschnitt 12 einen Außendurchmesser Dₚ₁, der kleiner ist als der äußere Durchmesser des zweiten rohrförmigen Abschnitts Dₚ₂.

In Figur 2 ist ein anmeldungsgemäßer Konnektor 30 schematisch dargestellt. Der Konnektor 30 umfasst einen flanschförmigen Konnektorabschnitt 32 und einen rohrförmigen Konnektorabschnitt 34, der sich entlang der Achse 31 von einem distalen, am flanschförmigen Abschnitt 32 angeordneten Ende zu einem proximalen Ende erstreckt. Der rohrförmige Konnektorabschnitt umfasst eine Wand 36 mit einer Innenseite 40, in welche beispielsweise der erste rohrförmige Abschnitt 12 der Blutpumpe 10 der Figur 1 eingeschoben werden kann. Weiterhin weist die Wand eine Außenseite 38 auf, wobei am proximalen Ende der Außenseite 38 eine Aufweitung 42 mit einem Rastnasenprofil angeordnet ist. Das Rastnasenprofil ist dabei so gestaltet, dass sich die radiale Breite bzw. der Durchmesser von einem proximalen Ende zu einem distalen Ende hin aufweitet, bis eine nicht näher dargestellte hintergreifende Fläche das Rastnasenprofil abschließt.

Die Innenseite 38 des Konnektors 30 besitzt einen Durchmesser Dₖ₁, welcher mit dem Einlassdurchmesser Dₚ₁ korrespondiert. Der flanschförmige Konnektorabschnitt 32 besitzt einen äußeren Durchmesser Dₖ₂ und kann so ausgebildet sein, dass er an seinem äußeren Rand beispielsweise Löcher zum Vernähen des Konnektors mit einem Organ aufweisen kann. Der Konnektor 30 kann beispielsweise einheitlich aus einem einzigen Metall oder einer Metallkomposition oder einem Kunststoff gebildet sein. Ferner kann beispielsweise der flanschförmige Abschnitt 32 im Bereich des rohrförmigen Konnektorabschnitts aus einem Metall bestehen, wohingegen der äußere Rand des flanschförmigen Abschnitts 32 aus einem flexiblen Material besteht, das leicht mit einem Organ vernäht werden kann. Der Durchmesser Dₖ₂ kann dabei derart gewählt sein, dass dieser größer ist als der Durchmesser Dₚ₂ des zweiten rohrförmigen Abschnitts 14 der Blutpumpe 10 der Figur 1. Hierdurch kann eine verbesserte Stabilität der Blutpumpe im mit dem Konnektor verbundenen Zustand erreicht werden.

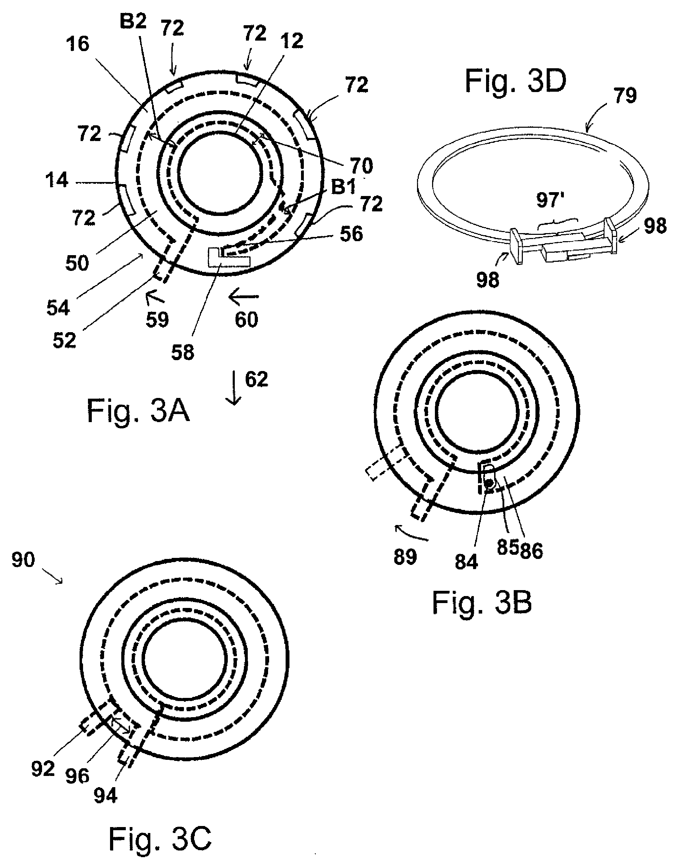

In den Figuren 3 ist eine Darstellung der Blutpumpe 10 in einer Ebene senkrecht zur Achse 18 dargestellt. Mit durchgezogenen Linien sind der erste rohrförmige Abschnitt 12, der zweite rohrförmige Abschnitt 14 sowie der dazwischenliegende flanschförmige Abschnitt 16 erkennbar. Gestrichelt eingezeichnet ist ein offener Sperrring 50, dessen Greifelement 52 sich radial bis außerhalb des zweiten rohrförmigen Abschnitts 14 erstreckt. Das Greifelement 52 ist an einem Greifende 54 des Sperrrings angeordnet. Der Sperrring erstreckt sich über ein Winkelsegment von mehr als 330° von seinem Greifende 54 bis zum offenen Ende 56, wo er an einem als Teil der Pumpe ausgebildeten Anschlag 58 anliegt. Der Anschlag 58 verhindert, dass beim Aufbringen einer Kraft auf das Greifelement in Richtung 59 der Sperrring 50 unbeschränkt in die Richtung 60 weitergedreht werden kann. Zudem verhindert der L-förmige Anschlag 58, dass der Sperrring in der radialen Richtung 62 ausbrechen kann. Der Sperrring 50 kann dabei lose, ohne stoff-, kraft- oder formschlüssig mit der Pumpe verbunden zu sein, in einem Führungsspalt liegen.

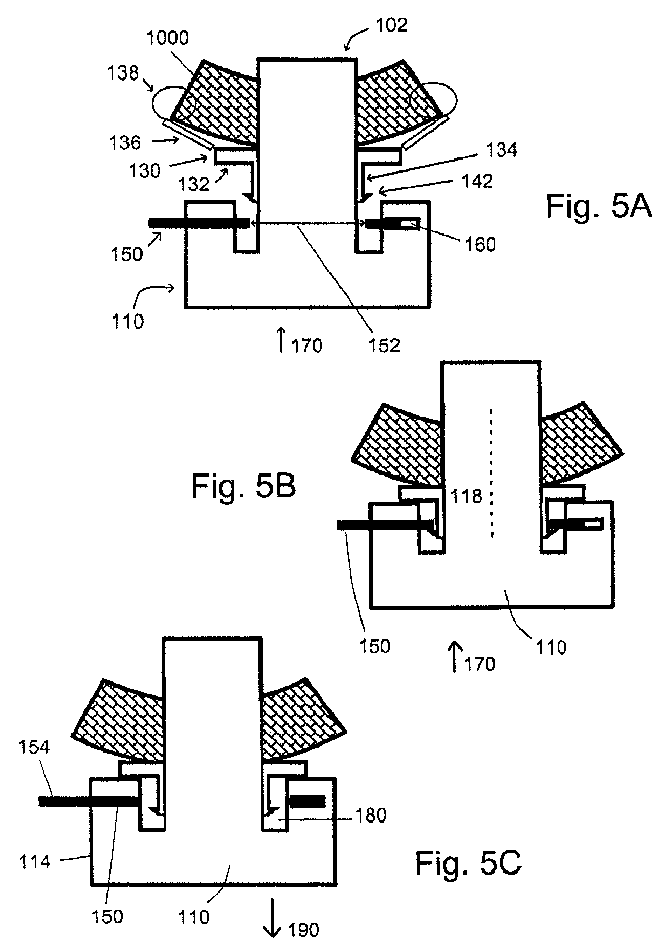

Zwischen dem rohrförmigen Abschnitt 12 und dem rohrförmigen Abschnitt 14 befindet sich im Bereich des flanschförmigen Abschnitts 16 eine Ausnehmung 70, die ebenfalls ringförmig ausgebildet ist und deren in radialer Richtung 62 betrachtete Breite im Wesentlichen der Breite der Aufweitung 42 mit Rastnasenprofil entspricht. Es ist erkennbar, dass der in seinem ersten, unbelasteten Zustand dargestellte Sperrring in die Ausnehmung 70 radial eingreift. Das Zusammenspiel zwischen der rastnasenförmigen Aufweitung 42 und dem Sperrring 50 wird anhand der Figuren 5 näher erläutert. In der Figur 3A sind zudem optionale Stoppelemente 72 dargestellt, welche an der Blutpumpe, radial außerhalb des Sperrrings, angeordnet sind. Die Stoppelemente verhindern, dass der Sperrring zu stark radial aufgedehnt werden kann, und können beispielsweise aus Silikon, Stahl oder Schaumstoff gefertigt sein. Diese Stoppelemente dienen auch einer zusätzlichen, äußeren Zentrierung und können auch bei den anderen Ausführungsbeispielen der Blutpumpe bzw. des Systems eingesetzt werden. Des Weiteren besitzt der Sperrring in der Umgebung (beispielsweise eines 60°-Segments, im Gegenuhrzeigersinn betrachtet) des Anschlags 58 eine verringerte radiale Breite B1 gegenüber einer Breite B2 des Sperrrings in anderen Segmenten. Hierdurch wird bewirkt, dass der Sperrring die rastnasenförmige Aufweitung auch im Bereich des Anschlags freigibt. Der Übergang der Breite des Sperrrings von der Breite B1 zur Breite B2 kann dabei, wie eingezeichnet, graduell, d. h. über ein bestimmtes Winkelsegment (beispielsweise 10°), oder als Sprung erfolgen. Der Übergang kann eine linear oder nichtlinear zunehmende Breite von der Breite B1 zur Breite B2 besitzen.

In Figur 3B ist ein alternativer Sperrring 80 dargestellt. Dieser ist im Wesentlichen identisch mit dem Sperrring 50 der Figur 3A, wird jedoch mittels eines mit der Pumpe verbundenen Stiftes 84 und einer dazu korrespondierenden Ausnehmung der Sperrrings 86 formschlüssig in dem Führungsspalt gehalten. Beim Aufbringen einer Kraft in Richtung 89 wird zum einen der Sperrring aufgeweitet, und der Sperrring dreht sich um den Stift 84. Dabei kann in einer vorteilhaften Ausgestaltung der Sperrring innerhalb eines vom Stift 84 gegen den Uhrzeigersinn gelegenen Winkelsegments von beispielsweise 30° bis 100°, vorzugsweise von 50° bis 80°, eine geringere Breite besitzen, so dass der Sperrring im Bereich des Winkelsegments nicht in die Ausnehmung 70 ragt. So wird vermieden, dass ein lediglich starkes Dehnen des Sperrrings die Rastnase des Konnektors freigibt. Damit der Sperrring 86 auch auf der Höhe des Stiftes 84 ausreichend aufgeweitet wird, wird der Ring am Stift 84 in einer Kulissenbahn 85 geführt, welche sich entlang der radialen Richtung des Sperrrings erstreckt. Wird zum Aufweiten eine Kraft in Richtung 89 auf den Sperrring ausgeübt, bewegt sich das Kulissenende des Sperrrings auf der Kulissenbahn 85 vom in der Fig. 3B gezeigten Zustand weg, bis es mit dem gegenüberliegenden Ende der Kulissenbahn am Stift 84 zur Anlage kommt.

Obgleich in den Figuren 3A und 3B der Sperrring ein offener Sperrring ist, d. h., dass er ein Winkelsegment von weniger als 360° umfasst, kann, wie beispielsweise in Figur 3C gezeigt, der Sperrring auch einen Winkelbereich von mehr als 360° umschließen. Der in Figur 3C dargestellte Sperrring 90 weist dabei ein erstes Greifelement 92 und ein zweites Greifelement 94 auf. Der zwischen den beiden Greifelementen liegende Bereich 96 wird dabei von einem links des Greifelements 94 liegenden Teil des Sperrrings und einem rechts mit dem Greifelement 92 verbundenen Teilsegment des Sperrrings gebildet, so dass der Sperrring in dem Bereich 96 zweilagig vorliegt. Durch Aufbringen von Druck auf die Greifelemente 92 und 94 so, dass diese aufeinander zu gedrückt werden, kann der Sperrring geweitet werden.

Ein weiteres Ausführungsbeispiel eines Sperrrings ist in der Fig. 3D dargestellt. Der Sperrring 97ist ähnlich dem Ausführungsbeispiel der Fig. 3C ausgebildet. Der Sperrring 97 besitzt einen Überlappungsbereich 97' und zwei Griffelemente 98. Durch Pressen der Griffelemente 98 gegeneinander wird der Sperrring aufgeweitet.

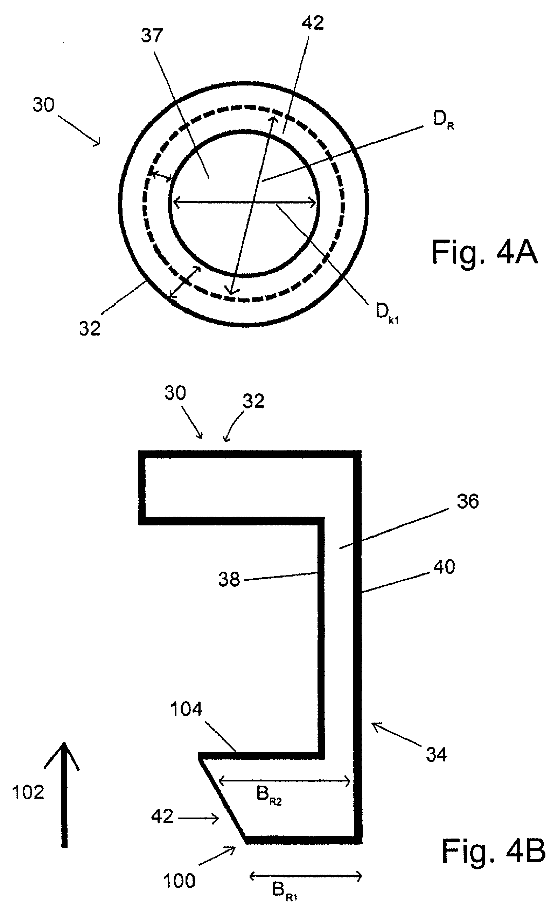

Der Konnektor 30 umfasst den flanschförmigen Abschnitt 32 und, wie in der Beschreibung der Figur 2 erwähnt, einen rohrförmigen Konnektorabschnitt 34. Ein durch die Wand 36 des rohrförmigen Abschnitts 34 begrenztes Lumen 37 ist in Figur 4A erkennbar. Das Lumen 37 besitzt einen Durchmesser Dₖ₁. Am proximalen Ende des rohrförmigen Konnektorabschnitts 34 befindet sich die rastnasenförmige Aufweitung 42. Deren radialer Durchmesser weitet sich von dem Durchmesser Dₖ₁ bis zu einem größeren Durchmesser Dᵣ auf.

Das Rastnasenprofil der Aufweitung ist in Figur 4B näher dargestellt. Der Konnektor 30 besitzt den flanschförmigen Konnektorabschnitt 32 sowie den rohrförmigen Konnektorabschnitt 34, der eine Wand 36 mit einer Außenseite 38 und einer Innenseite 40 aufweist. Die Innenseite 40 des rohrförmigen Abschnitts 34 weist in dem vorliegenden Beispiel keinerlei Profilierung auf. Die Aufweitung ist vollständig im Bereich der Außenseite der Wand 36 angeordnet. Die Aufweitung 42 umfasst eine Rastnase 100, deren Dicke in der Richtung 102 bis zur hintergreifenden Fläche 104 von einer ersten Breite Bᵣ₁ bis zu einer zweiten Wandbreite Bᵣ₂ zunimmt.

Alternative Profile des Rastnasenprofils sind ebenfalls möglich. So kann beispielsweise die Breite Bᵣ₁ im Wesentlichen gleich null gewählt werden, so dass das Rastnasenprofil an seinem proximalen Ende eine Spitze aufweist.

Das Zusammenwirken der Blutpumpe mit dem Sperrring und dem Konnektor soll anhand der Figuren 5A-C näher illustriert werden.

In Figur 5A ist unter anderem eine Herzwand 1000 gezeigt, in welche beispielsweise durch Stanzen eine Öffnung 102 eingebracht wurde. Eine Blutpumpe 110 wird mithilfe eines Konnektors 130 in die Öffnung 102 eingeführt. Dabei entspricht die Pumpe 110 im Wesentlichen einer Pumpe, wie sie in Details bereits in den Figuren 1 und 3 erläutert wurde. Der Konnektor 130 entspricht im Wesentlichen einem Konnektor 30, wie er in Figur 2 gezeigt ist. So umfasst der Konnektor 130 ebenfalls einen flanschförmigen Abschnitt 132 sowie einen rohrförmigen Konnektorabschnitt 134 mit einer Aufweitung 142. Am radialen Rand des flanschförmigen Abschnitts 132 befindet sich zusätzlich ein flexibler Ring, der stoffschlüssig mit dem flanschförmigen Abschnitt 132 verbunden ist. Der Ring 136 ist mithilfe von Nähten 138 mit der Herzwand 100 vernäht. Die Blutpumpe 110 umfasst unter anderem einen Sperrring 150, der in einem Führungsspalt 160 geführt ist. In dem in Figur 5A dargestellten Zustand wird die Blutpumpe 110 in axialer Richtung 170 in den rohrförmigen Konnektorabschnitt eingeschoben. Es ist erkennbar, dass der Innendurchmesser 152 des Sperrrings im unbelasteten Zustand kleiner ist als der größte Außendurchmesser der Aufweitung 142. Wird nun die Blutpumpe 110 in Richtung 170 auf den Konnektor 130 aufgeschoben, trifft der Sperrring auch die Aufweitung und wird graduell durch die im Durchmesser größer werdende Aufweitung aufgeweitet, bis das distale Ende der Rastnase erreicht ist und der Sperrring, wie in Figur 5B gezeigt ist, die Rastnase hintergreift. In diesem die Rastnase hintergreifenden Zustand ist der Sperrring vorzugsweise unbelastet, d. h., er nimmt seine ohne aufgebrachte Spannung gegebene Form an.

Es ist für den Fachmann deutlich ersichtlich, dass die Blutpumpe 110 in dem in Figur 5B dargestellten ersten, vorzugsweise unbelasteten Zustand des Sperrrings um die Achse 118 drehbar gelagert ist. Um die Blutpumpe 110 beispielsweise zu explantieren, kann durch das radial aus dem zweiten rohrförmigen Abschnitt 114 herausstehende Greifelement 154 des Sperrrings 150 eine Kraft aufgebracht werden, so dass der Sperrring 150 so aufgeweitet wird, dass der in der Ausnehmung 180 liegende Abschnitt des rohrförmigen Konnektorabschnitts 134 freigegeben wird bzw. die Aufweitung nicht länger hintergriffen wird und die Pumpe 110 in der axialen Richtung 190 explantiert werden kann. Dies wird in Figur 5C illustriert.

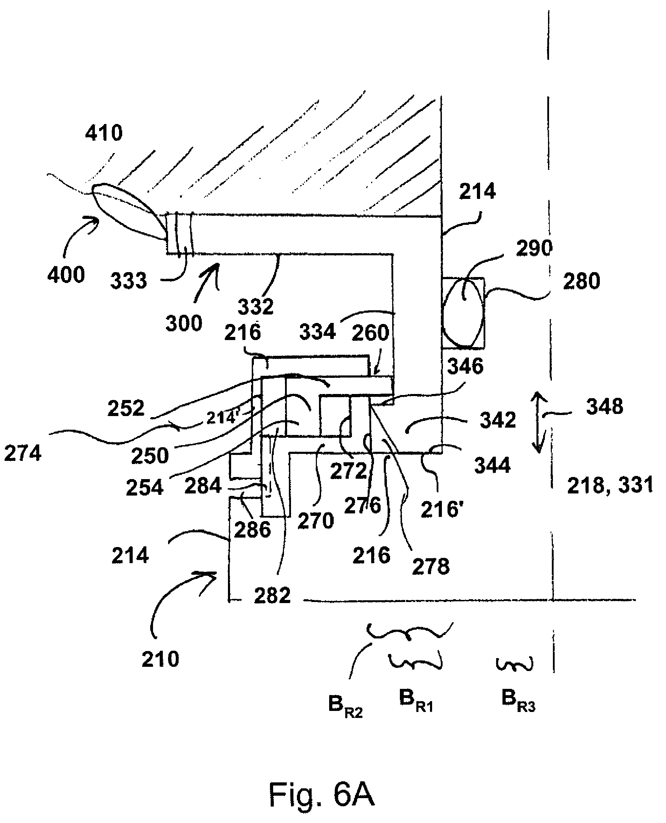

In Figur 6A ist ein weiteres Ausführungsbeispiel eines VAD-Konnektor-Systems dargestellt. Dabei wird lediglich ein Profilausschnitt der Achsen 218 der Blutpumpe bzw. 331 des Konnektors nach radial außen gezeigt. Das System umfasst eine Blutpumpe 210 und einen Konnektor 300. Der Konnektor 300 entspricht dabei im Wesentlichen den bereits diskutierten Konnektoren 30 bzw. 130. Der Konnektor umfasst einen flanschförmigen Abschnitt 332, der Öffnungen 333 umfasst, durch die eine Naht 400 geführt werden kann, um den flanschförmigen Konnektorabschnitt 332 mit einer Herzwand 410 zu verbinden. Der rohrförmige Abschnitt 334 des Konnektors 300 besitzt an seinem proximalen Ende eine Aufweitung 342 in Form einer Rastnase, die an seinem proximalsten Ende einen ebenen Abschnitt 344 umfasst, der eine Breite Bᵣ₁ besitzt und der sich in distaler Richtung gehend über eine Höhe 348 bis zu einer Breite Bᵣ₂ aufweitet. Am distalen Ende der Rastnase befindet sich eine zu hintergreifende Fläche, die eine Breite von Bᵣ₃ besitzt, die der Differenz Bᵣ₂ - Bᵣ₁ entspricht. An der Innenwand des rohrförmigen Abschnitts 334 befindet sich keinerlei Profilierung.

Die Blutpumpe 210 umfasst einen ersten rohrförmigen Abschnitt 212, der eine Nut 280 umfasst, in welcher ein Dichtring 290 angeordnet ist, um eine Dichtung zwischen der Innenwand des rohrförmigen Konnektorabschnitts 334 und dem ersten rohrförmigen Abschnitt 214 zu bilden. Vom flanschförmigen Abschnitt 216 bis zum zweiten rohrförmigen Abschnitt 214 der Blutpumpe 210 erstreckt sich radial unter anderem ein Sperrring 250, der in einem Führungsspalt 260 geführt ist und so konfiguriert ist, dass er im unbelasteten Zustand die zu hintergreifende Fläche 346 hintergreift und mittels eines Mechanismus wie beispielsweise in der Beschreibung der Figuren 5 erläutert aufgeweitet werden kann, so dass die Blutpumpe 210 vom Konnektor 300 abgezogen werden kann. Der Führungsspalt 260 nimmt einen ersten Abschnitt 252 des Sperrrings 250 auf, wobei die Breite des Führungsspalts in axialer Richtung des Führungsspalts 260 so gewählt ist, dass die Breite im Wesentlichen der Höhe des Abschnitts 252 in axialer Richtung entspricht, so dass der Sperrring 250 kein axiales Spiel besitzt.

Des Weiteren umfasst der Sperrring 250 einen abgewinkelten Bereich 254, der an einer Anschlagsfläche 272 eines Anschlagrings 270 radial gehalten werden kann. Dem Anschlag 272 radial gegenüber außen liegend befindet sich ein äußerer radialer Anschlag 274 des flanschförmigen Abschnitts 116 der Blutpumpe. Zwischen einem radial inneren Bereich des Anschlagrings 276 und dem ersten rohrförmigen Abschnitt 214 ist eine Ausnehmung 278 vorhanden, die eine Breite Bᵣ₂ aufweist, die im Wesentlichen mit der Breite des größten Durchmessers der Aufweitung 342 korrespondiert. Am proximalen Ende besitzt die Aufweitung 342 einen ebenen Abschnitt 344, der mit einem ebenen Abschnitt 216' des flanschförmigen Abschnitts 216 korrespondiert. Zwischen dem Sperrring 250 und einer Wandung 214' der Blutpumpe befindet sich eine Kavität 282, die sich bei längerem Verbleib der Blutpumpe im organischen Gewebe mit Gewebe verfüllen kann. Damit dieses Gewebe bei der Explantierung nicht die Beweglichkeit des Sperrrings einschränkt, ist vorgesehen, im Anschlagring 270 Kanäle 284 einzubringen, die in Öffnungen 286 des zweiten rohrförmigen Abschnitts 214 münden.

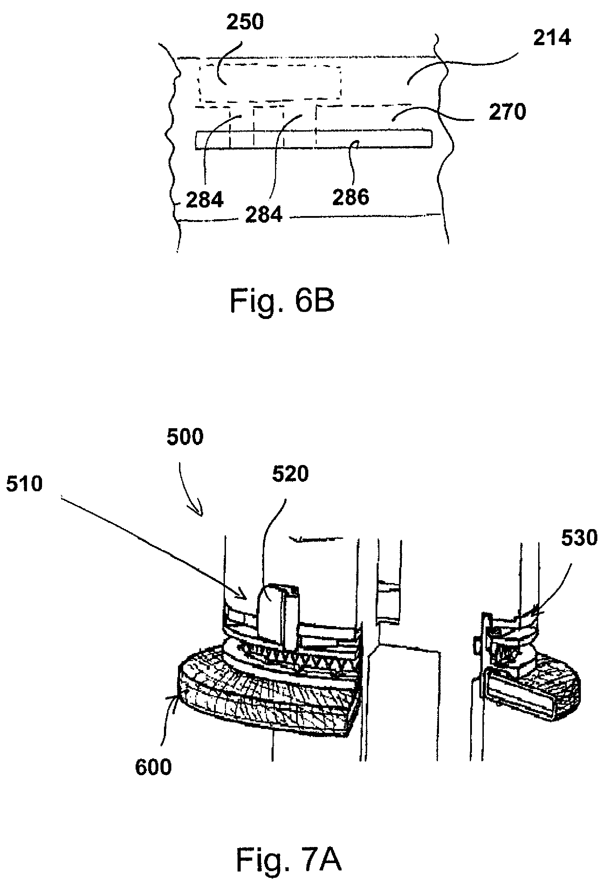

Dies ist in Figur 6B nochmals in einer Ansicht dargestellt. Der rohrförmige Abschnitt 214 besitzt eine Öffnung 286, wobei der Anschlagring 270 Kanäle 284 besitzt, durch die in der Kammer 282 befindliches Material beim Aufweiten des Sperrrings in radialer Richtung ausgestoßen werden kann. Auf diese Weise wird verhindert, dass der Sperrring im eingewachsenen Zustand blockiert ist.

In den Figuren 7A-C ist eine weitere Alternative eines VAD-Konnektor-Systems dargestellt.

Die Blutpumpe 500 wird mit einem Konnektor 600 verbunden. Die Blutpumpe umfasst dabei einen Sperrring 510, der eine Greiffläche 520 besitzt. Der Anschlagring 530 wurde dabei in einen Führungsspalt eingesetzt und definiert einen Führungsspalt, der im Wesentlichen der Höhe des Sperrrings entspricht. Der Anschlagring wurde dabei mit der Pumpe nachträglich verschweißt.

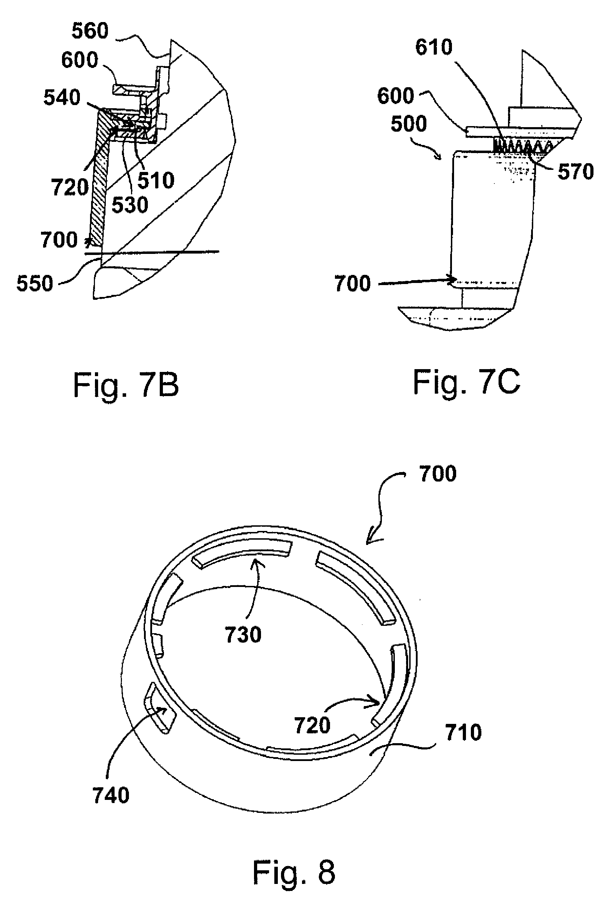

Ein Ausschnitt der in der Figur 7A dargestellten Pumpe ist in Figur 7B dargestellt. Neben der Blutpumpe 500 und dem Konnektor 600 ist zudem ein über die Blutpumpe stülpbarer Silikonmantel 700 dargestellt, dessen Funktionalität noch beschrieben wird.

Die Blutpumpe 500 umfasst einen flanschförmigen Abschnitt 540, welcher einen gegenüber dem zweiten rohrförmigen Abschnitt 550 vergleichbaren Radius besitzt und unter anderem den bereits erwähnten Sperrring und Anschlagring umfasst. Alternativ kann der Radius des flanschförmigen Abschnitts 540 kleiner als der Radius des zweiten rohrförmigen Abschnitts gewählt sein. Der flanschförmige Abschnitt 540 ist lediglich größer als der erste rohrförmige Abschnitt 560 gewählt. Der Silikonmantel 700 ist im Detail in Figur 8 dargestellt.

In Figur 7C sind zudem ein an der Blutpumpe angeordneter, gezahnter Abschnitt 570 und ein am Konnektor angeordneter, mit dem Abschnitt 570 korrespondierender gezahnter Abschnitt 610 dargestellt. Im dargestellten ersten Zustand (d. h., die Blutpumpe ist in den Konnektor eingeführt) sind die Zähne des Abschnitts 570 mit den Zähnen des Abschnitts 610 in Eingriff und verhindern, dass die Blutpumpe gegenüber dem Konnektor verdreht werden kann, d. h., Blutpumpe und Konnektor sind miteinander drehfest verbunden. Wird die Blutpumpe gegenüber dem dargestellten Zustand um die Höhe der Zähne aus dem Konnektor gezogen, ist die Blutpumpe wieder drehbar, und es kann eine andere Orientierung der Blutpumpe gegenüber dem Konnektor eingestellt werden.

Im vorliegenden Beispiel sind sowohl der gezahnte Abschnitt 570 als auch der gezahnte Abschnitt 610 kreisförmig ausgebildet. Hierdurch kann jede beliebige Orientierung der Blutpumpe gegenüber dem Konnektor eingestellt werden und die Orientierung drehfest beibehalten werden.

Die gezahnten Abschnitte können beispielsweise aus einem biokompatiblen Werkstoff mit einem Metall oder einem Kunststoff gebildet sein. Die Zähne sind vorzugsweise inflexibel bzw. weisen eine ausreichende Härte auf, so dass ein Verdrehen der Abschnitte gegeneinander nicht möglich ist.

In Figur 8 ist der Silikonmantel 700 dargestellt. Dieser umfasst eine erste Mantelfläche 710, welche auf dem zweiten rohrförmigen Abschnitt 550 zum Liegen kommt. Ferner umfasst der Silikonmantel mehrere Zungen 720 bzw. 730, welche in Öffnungen des flanschförmigen Abschnitts 540 eingreifen (siehe Figur 7B). Durch das Eingreifen der Zungen kann Gewebe nicht vollständig in die Öffnungen dringen und die Öffnungen füllen, sondern lediglich in sehr geringem Umfang in die Spalte zwischen den Zungen und den Rändern der Öffnungen einwachsen, so dass der Sperrring bei einer Explantation der Pumpe trotz eingewachsenen Gewebes ein Lösen der Blutpumpe vom Konnektor ermöglicht.

Unabhängig von den Zungen kann der Silikonmantel 700 eine Öffnung 740 umfassen, durch welche ein Griff des Sperrrings hindurchragen kann.

## Patentansprüche

1. System zum Verbinden einer Blutpumpe mit einem Herzen, umfassend:
eine Blutpumpe (10) zur Förderung von Blut, wobei die Blutpumpe einen ersten rohrförmigen Abschnitt (12) und einen zweiten rohrförmigen Abschnitt (14) umfasst und zwischen dem ersten und dem zweiten rohrförmigen Abschnitt ein flanschförmiger Abschnitt (16) vorhanden ist;
einen Konnektor (30) mit einem sich zwischen einem distalen Ende und einem proximalen Ende in einer axialen Richtung erstreckenden rohrförmigen Konnektorabschnitt (34) mit einem Lumen zur Aufnahme des ersten rohrförmigen Abschnitts der Blutpumpe und mit einem am distalen Ende angeordneten flanschförmigen Konnektorabschnitt (32) zur Befestigung des Konnektors an einem Organ, wobei eine das Lumen umlaufende Wand des ersten rohrförmigen Konnektorabschnitts eine die Wand zumindest abschnittsweise umlaufende Aufweitung (42) mit einer Rastnase (100) aufweist;
wobei im flanschförmigen Abschnitt der Blutpumpe ein in einem Führungsspalt aufweitbarer Sperrring (50) angeordnet ist, welcher derart im Führungsspalt geführt und konfiguriert ist, dass der Sperrring in einem ersten Zustand die Aufweitung der Wand hintergreift und in einem zweiten, aufgeweiteten Zustand die Wand nicht hintergreift.

2. System nach Anspruch 1, wobei die Aufweitung die Wand vollständig umläuft.

3. System nach einem der vorhergehenden Ansprüche, wobei die Rastnase an einem proximalen Ende bevorzugt einen ebenen Abschnitt umfasst, welcher mit dem flanschförmigen Abschnitt der Blutpumpe korrespondiert.

4. System nach einem der vorhergehenden Ansprüche, wobei der Führungsspalt eine Breite besitzt, die mit einer Höhe des Sperrrings korrespondiert, so dass die Blutpumpe im ersten Zustand in axialer Richtung fixiert ist.

5. System nach einem der vorhergehenden Ansprüche, wobei die Blutpumpe im ersten Zustand um die axiale Richtung drehbar gehalten ist.

6. System nach einem der Ansprüche 1 bis 4, wobei die Blutpumpe einen ersten verzahnten Abschnitt und der Konnektor einen zweiten, mit dem ersten korrespondierenden, verzahnten Abschnitt umfasst, so dass die Blutpumpe im ersten Zustand im Konnektor drehfest gehalten ist.

7. System nach einem der vorhergehenden Ansprüche, wobei der Führungsspalt in einer radialen Richtung offen ist.

8. System nach einem der vorhergehenden Ansprüche, wobei ein Abschnitt des flanschförmigen Abschnitts der Blutpumpe einen, den Sperrring im zweiten Zustand vorzugsweise zentrierenden Anschlagsring umfasst.

9. System nach einem der vorhergehenden Ansprüche, wobei der Sperrring einen die Aufweitung hintergreifenden Abschnitt und einen weiteren, zum hintergreifenden Abschnitt abgewinkelten Abschnitt aufweist, welcher derart ausgebildet ist, dass der abgewinkelte Abschnitt im ersten Zustand an einem Teilsegment des flanschförmigen Abschnitts oder des ersten Rohrabschnitts radial abgestützt ist.

10. System nach einem der vorhergehenden Ansprüche, wobei der Sperrring an einem Ende an der Blutpumpe fixiert ist.

11. System nach einem der vorhergehenden Ansprüche, wobei der Sperrring ein offener Sprengring ist.

12. System nach einem der vorhergehenden Ansprüche, wobei der Sperrring ein Greifelement zur Überführung des Systems vom ersten in den zweiten Zustand aufweist, wobei die Blutpumpe vorzugsweise ein zum Greifelement korrespondierendes Element aufweist, so dass eine auf das Greifelement in Richtung des korrespondierenden Elements aufgebrachte Kraft den Sperrring aufweitet.

13. System nach einem der vorhergehenden Ansprüche, wobei der flanschförmige Abschnitt der Blutpumpe Öffnungen zum zweiten rohrförmigen Abschnitt aufweist.

14. System nach Anspruch 13, wobei ein auf die Blutpumpe stülpbarer Mantel vorhanden ist, welcher mindestens eine Zunge umfasst, die in die mindestens eine Öffnung ragt.

15. System nach einem der vorhergehenden Ansprüche, wobei der erste rohrförmige Abschnitt eine Nut aufweist, in welcher ein Dichtelement derart angeordnet ist, dass das Dichtelement an einer Innenseite der Wand dichtend anliegt.

16. System zum Verbinden einer Blutpumpe mit einem Herzen, umfassend:
eine Blutpumpe (10) zur Förderung von Blut, wobei die Blutpumpe einen ersten rohrförmigen Abschnitt (12) und einen zweiten rohrförmigen Abschnitt (14) umfasst und zwischen dem ersten und dem zweiten rohrförmigen Abschnitt ein flanschförmiger Abschnitt (16) vorhanden ist;
einen Konnektor (30) mit einem sich zwischen einem distalen Ende und einem proximalen Ende in einer axialen Richtung erstreckenden rohrförmigen Konnektorabschnitt (34) mit einem Lumen zur Aufnahme des ersten rohrförmigen Abschnitts der Blutpumpe und mit einem am distalen Ende angeordneten flanschförmigen Konnektorabschnitt (32) zur Befestigung des Konnektors an einem Organ;
wobei die Blutpumpe einen ersten, gezahnten Abschnitt und der Konnektor einen zweiten, mit dem ersten Abschnitt korrespondierenden gezahnten Abschnitt umfasst, so dass die Blutpumpe gegenüber dem Konnektor drehfest ist, wenn der erste und der zweite Abschnitt ineinandergreifen.

17. Mantel mit einer ersten Mantelfläche und mindestens einer von der Mantelfläche nach innen ragenden Zunge zur Verwendung in einer Blutpumpe zur Förderung von Blut, wobei die Blutpumpe einen ersten rohrförmigen Abschnitt und einen zweiten rohrförmigen Abschnitt umfasst und zwischen dem ersten und dem zweiten rohrförmigen Abschnitt ein flanschförmiger Abschnitt vorhanden ist;
wobei im flanschförmigen Abschnitt der Blutpumpe ein in einem Führungsspalt aufweitbarer Sperrring angeordnet ist, welcher im Führungsspalt geführt und konfiguriert ist und mit mindestens einer Zunge in einen Abschnitt des Führungsspalts oder eine zum Führungsspalt führende Öffnung eingreift.

18. System nach einem der vorhergehenden Ansprüche, wobei der erste rohrförmige Abschnitt ein Einlassstutzen einer Blutpumpe ist.
